# FASCICULE DE BREVET EUROPEEN

(11) **EP 2 860 172 B1**
(45) Date de publication et mention de la délivrance du brevet: **21.06.2017**
(21) Numéro de dépôt: 14188892.5
(22) Date de dépôt: 14.10.2014
(51) Int. Cl.: C07C 231/10, C07C 231/24

(54) **Procédé de préparation de dérivés d'acétamidophényle**
Verfahren zur Herstellung von Acetamidophenylderivaten
Method for preparing acetamidophenyl

(30) Priorité: 14.10.2013 FR 1359972
(43) Date de publication de la demande: 15.04.2015
(73) Titulaire: Minakem, 59640 Dunkerque (FR)
(72) Inventeur: LeMaire, Marc, Lionel, 69100 Villeurbanne (FR); Joncour, Roxan, 69100 Villeurbanne (FR); Duguet, Nicolas, Fabien, Denis, 69330 Meyzieu (FR); Metay, Estelle, Catherine, Danielle, 69120 Vaulx en Velin (FR); Ferreira, Amadeo, 59500 Douai (FR)
(74) Mandataire: Lavoix

(56) Documents cités:
- US-A- 5 856 575
- V. Merz ET AL: "Ueber die Darstellung von Aminen aus Phenolen und Alkoholen", Berichte der Deutschen Chemischen Gesellschaft, 1 janvier 1881 (1881-01-01), pages 2343-2347, XP055124084, Extrait de l'Internet: URL:http://onlinelibrary.wiley.com/store/1 0.1002/cber.188101402165/asset/18810140216 5_ftp.pdf?v=1&t=hwknms2c&s=9eaac877cfdf2e7 5a5f96ee090ae2416cdbc1c89 [extrait le 2014-06-18]
- ROXAN JONCOUR ET AL: "Amidation of phenol derivatives: a direct synthesis of paracetamol (acetaminophen) from hydroquinone", GREEN CHEMISTRY, vol. 16, no. 6, 1 juin 2014 (2014-06-01), page 2997, XP055167214, ISSN: 1463-9262, DOI: 10.1039/c4gc00166d

## Description

La présente invention concerne un procédé de préparation de N-acétyl-p-aminophénol (également nommé paracétamol ou acétaminophène).

Le N-acétyl-p-aminophénol est un des médicaments les plus consommés dans le monde, avec une consommation mondiale de 115 000 T/an. En France, le doliprane, le dafalgan et l'efferalgan sont les trois médicaments les plus consommés en quantités et sont à base de N-acétyl-p-aminophénol. Cependant, l'ancienneté et la simplicité de la méthode de synthèse du N-acétyl-p-aminophénol en font un produit aux coûts de production particulièrement faibles (entre 2 et 3 euros par kilo de N-acétyl-p-aminophénol pur). Ceci fait que le premier médicament contenant du N-acétyl-p-aminophénol ne se classe qu'à la huitième place des médicaments les plus consommés en valeur.

Divers procédés de préparation de N-acétyl-p-aminophénol sont décrits dans la littérature.

En particulier, le brevet US 5,856,575 décrit un procédé de préparation de N-acétyl-p-aminophénol à partir d'hydroquinone et d'acétamide en présence d'un catalyseur solide de type hétéropolyacide ou d'un de ses sels. La préparation du N-acétyl-p-aminophénol par réaction de l'acétamide avec l'hydroquinone en présence d'un sel de césium de l'acide phosphotungstique à 280-300°C est exemplifiée avec une conversion de l'hydroquinone de 95%wt et une sélectivité de 86% en N-acétyl-p-aminophénol. L'acétamide peut être généré *in situ* à partir d'acétate d'ammonium. La préparation du N-acétyl-p-aminophénol par réaction d'acétate d'ammonium et d'hydroquinone en présence d'un sel de césium de l'acide silicotungstique à 300°C est exemplifiée avec une conversion de l'hydroquinone de 88%wt et une sélectivité de 77% en N-acétyl-p-aminophénol.

On cherche à développer un procédé de préparation de N-acétyl-p-aminophénol :
- conduisant à un rendement amélioré, et/ou
- moins coûteux, et/ou
- réalisable dans des conditions plus écologiques, et/ou
- réalisable dans des conditions rendant le procédé plus facilement industrialisable.

A cet effet, l'invention fournit un procédé de préparation de N-acétyl-p-aminophénol comprenant une étape de réaction de l'hydroquinone avec l'acétate d'ammonium, la réaction étant réalisée en l'absence d'hétéropolyacide ou d'un de ses sels.

Les inventeurs ont découvert que, lorsque de l'acétate d'ammonium est utilisé comme réactif, la réaction avec l'hydroquinone pour former le N-acétyl-p-aminophénol peut être réalisée en l'absence de catalyseur de type hétéropolyacide. Comme le procédé selon l'invention ne nécessite pas de catalyseur hétéropolyacide, il est moins coûteux et plus écologique que celui décrit dans le brevet US 5,856,575.

Sans vouloir être lié à une théorie particulière, il semblerait que l'acétate d'ammonium forme un milieu de type liquide ionique, ce qui faciliterait sa réaction avec l'hydroquinone pour former le N-acétyl-p-aminophénol.

Le procédé est donc réalisé en l'absence d'hétéropolyacide ou d'un de ses sels. Par « hétéropolyacide », on entend un composé comprenant :
- un métal, tel que du tungstène, du molybdène ou du vanadium,
- de l'oxygène,
- un élément du bloc p du tableau périodique, tel que du silicium, du phosphore ou de l'arsenic,
- un hydrogène acide.

On peut par exemple citer les hétéropolyacides suivants:
- H₄Xⁿ⁺M₁₂O₄₀, X = Si, Ge; M = Mo, W
- H₃Xⁿ⁺M₁₂O₄₀, X = P, As; M = Mo, W
- H₆X₂M₁₈O₆₂, X=P, As; M = Mo, W
et en particulier l'acide silicotungstique (H₄W₁₂SiO₄₀), l'acide phosphotungstique (H₃PW₁₂O₄₀), l'acide phosphomolybdique (H₃PMo₁₂O₄₀) et l'acide vanadotungstique. Comme sel d'hétéropolyacide, on peut citer les sels d'ammonium d'hétéropolyacide ou de métal alcalin d'hétéropolyacide, par exemple de potassium ou de césium.

Dans le procédé selon l'invention, la réaction entre l'hydroquinone et l'acétate d'ammonium peut être réalisée en l'absence de solvant ou en présence de solvant.

Lorsqu'elle est mise en oeuvre en l'absence de solvant, c'est en fait l'acétate d'ammonium qui sert de solvant. De préférence, lorsque la réaction est mise en oeuvre en l'absence de solvant, au moins 2, notamment au moins 3, de préférence au moins 10 équivalents d'acétate d'ammonium sont utilisés par rapport à l'hydroquinone. Lorsque l'on utilise moins de deux équivalents d'acétate d'ammonium par rapport à l'hydroquinone, la sélectivité en N-acétyl-p-aminophénol est généralement moins bonne. En effet, le milieu est alors plus concentré en hydroquinone et en N-acétyl-p-aminophénol, ce qui favorise des réactions secondaires indésirables, notamment la formation de diarylamines.

Lorsque la réaction est mise en oeuvre dans un solvant, le solvant est de préférence de l'acide acétique. Typiquement, de 1 à 10 équivalents, de préférence de 1 à 5 équivalents d'acide acétique par rapport à l'hydroquinone sont utilisés. L'acide acétique permet en effet d'acétyler le para-aminophénol, qui est un produit secondaire formé lors de la réaction, pour former le N-acétyl-p-aminophénol attendu. L'ajout d'acide acétique permet donc d'améliorer la sélectivité en N-acétyl-p-aminophénol. Il est alors possible de diminuer le nombre d'équivalents d'acétate d'ammonium par rapport à l'hydroquinone tout en conservant de bonnes conversions de l'hydroquinone et de bonnes sélectivités en N-acétyl-p-aminophénol. Ainsi, lorsque la réaction est mise en oeuvre en présence d'acide acétique, au moins 1,1, notamment au moins 1,2, de préférence au moins 2 équivalents d'acétate d'ammonium sont utilisés par rapport à l'hydroquinone. Généralement, de 1 à 5 équivalents, notamment de 2 à 4 équivalents, de préférence 2,5 équivalents d'acide acétique par rapport à l'acétate d'ammonium sont utilisés. Au-delà de 5 équivalents d'acide acétique par rapport à l'acétate d'ammonium, l'acétate d'ammonium est plus dilué dans le milieu et la conversion de l'hydroquinone est généralement plus faible. De préférence, le rapport molaire hydroquinone / acétate d'ammonium / acide acétique est de 1/2/5.

Dans le procédé, la réaction de l'hydroquinone avec l'acétate d'ammonium peut être réalisée en l'absence d'eau ou en présence d'eau. La présence d'eau n'est pas gênante pour la réaction Généralement, lorsque de l'eau est utilisée, de 0,1 à 20 équivalents, notamment de 1 à 15 équivalents, de préférence de 1 à 10 équivalents d'eau par rapport à l'hydroquinone peuvent être utilisés.

La réaction du procédé selon l'invention est typiquement réalisée à une température de 150°C à 300°C, notamment de 200 à 250°C, de préférence de 215 à 230°C. Ces températures sont en effet adaptées pour obtenir une bonne conversion de l'hydroquinone. Ces températures sont inférieures à celles mises en oeuvre dans les exemples du brevet US 5,856,575, ce qui est un avantage en termes de coût du procédé selon l'invention.

La réaction du procédé selon l'invention est généralement réalisée à une pression autogène, la pression étant alors généralement de 1 à 30 bars, de préférence de 10 à 20 bars.

La réaction du procédé selon l'invention dure typiquement de 1 à 24 heures, par exemple de l'ordre de 15 heures.

Le procédé peut comprendre une étape ultérieure de récupération du N-acétyl-p-aminophénol.

Il est possible de récupérer le N-acétyl-p-aminophénol par des méthodes de purification usuelles, par exemple par chromatographie, notamment par chromatographie sur colonne de gel de silice.

Selon une autre alternative, l'étape de récupération comprend les étapes de :
a) ajout d'eau au milieu réactionnel obtenu à la fin de la réaction, ce par quoi le N-acétyl-p-aminophénol précipite,
b) filtration du N-acétyl-p-aminophénol,
c) éventuellement recristallisation du N-acétyl-p-aminophénol dans l'eau.

Lorsque la réaction a été réalisée en présence d'acide acétique, l'étape de récupération comprend de préférence, avant l'étape a) d'ajout d'eau au milieu réactionnel, une étape α) d'évaporation de l'acide acétique du milieu réactionnel obtenu à la fin de la réaction. Le N-acétyl-p-aminophénol est plus soluble dans l'acide acétique que dans l'eau. La solubilité du N-acétyl-p-aminophénol dans l'acide acétique à 30 °C est de 87 g/L tandis qu'elle n'est que de 17 g/L dans l'eau à 30 °C. L'étape α) permet de minimiser le passage du N-acétyl-p-aminophénol dans la phase aqueuse lors de sa précipitation par ajout d'eau, et donc d'améliorer le rendement. L'acide acétique évaporé peut être récupéré et recyclé dans la réaction de l'hydroquinone avec l'acétate d'ammonium.

A la fin de l'étape de filtration b), on obtient un filtré (le N-acétyl-p-aminophénol) et un filtrat qui est une solution aqueuse comprenant principalement l'hydroquinone (n'ayant pas réagi lors de la réaction), de l'acétate d'ammonium (n'ayant pas réagi lors de la réaction), de l'acétamide (produit secondaire formé lors de la réaction), des traces de N-acétyl-p-aminophénol (n'ayant pas précipité lors de l'étape a)) et des traces de diarylamines (produits secondaires formés lors de la réaction).

Le procédé peut comprendre après l'étape de filtration b) des étapes de :
d) lavage du filtrat obtenu à l'étape b) avec un solvant apolaire (par exemple du cyclohexane, du n-hexane, du pentane, de l'éther de pétrole, du 1,4-dioxane, du diéthyl éther, du toluène, du chloroforme ou du dichlorométhane) (ce par quoi les traces de diarylamines sont extraites du filtrat),
e) évaporation de l'eau du filtrat pour obtenir un résidu (comprenant principalement l'hydroquinone, de l'acétate d'ammonium, de l'acétamide et des traces de N-acétyl-p-aminophénol),
f) introduction du résidu obtenu dans le milieu réactionnel lors de la réaction de l'hydroquinone avec l'acétate d'ammonium.

Il est ainsi possible de recycler l'hydroquinone et l'acétate d'ammonium n'ayant pas réagi lors de la réaction. De plus, lorsque la réaction de l'hydroquinone et de l'acétate d'ammonium est réalisée en présence d'eau, à des températures de 180°C à 250°C, l'acétamide présent dans le milieu réactionnel (du fait de l'introduction du résidu dans le milieu réactionnel) est hydrolysé en acétate d'ammonium. Ainsi, l'acétamide est transformé en acétate d'ammonium qui réagit avec l'hydroquinone pour former le N-acétyl-p-aminophénol. Par conséquent, le recyclage répété du résidu obtenu à l'étape e) dans la réaction ne conduit donc pas à un enrichissement en acétamide.

De préférence, la réaction du procédé selon l'invention est réalisée en l'absence de catalyseur métallique, ce qui est un avantage car les traces de catalyseur métallique doivent être évités lors de la préparation de médicaments ou de leurs intermédiaires de synthèse. Par catalyseur métallique, on entend un catalyseur hétéropolyacide ou un catalyseur à base d'un métal de transition.

Le procédé est de préférence réalisé par lots (« batch »).

Le procédé est illustré dans l'exemple qui suit.

### Exemple : Préparation de N-acétyl-p-aminophénol à partir d'hydroquinone

Excepté pour la réaction à plus grande échelle détaillée ci-après, les réactions des exemples ont été réalisées selon le protocole suivant.

La réaction a été réalisée dans un réacteur acier d'une capacité de 30 mL, muni d'une cuve en verre. L'hydroquinone et l'acétate d'ammonium ont été introduits dans la cuve puis l'acide acétique a éventuellement été ajouté. Le réacteur a été fermé et purgé sous flux d'argon pendant 10 min. Le réacteur a alors été chauffé. La température était suivie directement dans le réacteur. Une fois les 160 °C atteints, la réaction a été mise sous agitation (500 tr/min). Lorsque la température désirée a été atteinte, la réaction a été laissée 15 h sous agitation. Après réaction, le réacteur a été refroidi jusqu'à température ambiante (environ 25°C) puis le milieu a été dissous dans un mélange eau / méthanol (2/1).
Les conversions et les sélectivités indiquées sont calculées à partir de dosages HPLC effectués sur les milieux réactionnels avec les paramètres HPLC suivants :
- Type de colonne : C18
- Détecteur : UV ; longueurs d'ondes utilisées : 254 et 290 nm
- Eluant : eau/acétonitrile + 0,1% d'H₃PO₄, en gradient d'élution
- Débit éluant : 1 mL/min
Des étalons externes ont été réalisés suivants ces conditions afin de calculer précisément les quantités d'hydroquinone restant et de produits formés (N-acétyl-p-aminophénol et para-aminophénol (produit secondaire principal)) après chaque réaction.
Dans les tableaux qui suivent, les ratios indiqués sont des ratios molaires.

Dans les exemples qui suivent, l'hydroquinone est désignée « HQ », le N-acétyl-p-aminophénol est désigné « APAP » et le para-aminophénol est désigné PAP, comme illustré sur le schéma réactionnel suivant.

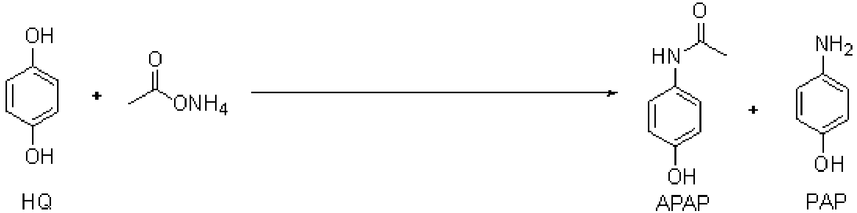

### • Réaction en l'absence d'acide acétique - Influence de la température et de la présence d'eau sur la conversion et la sélectivité

10 équivalents d'acétate d'ammonium par rapport à l'hydroquinone ont été utilisés dans ces expériences.

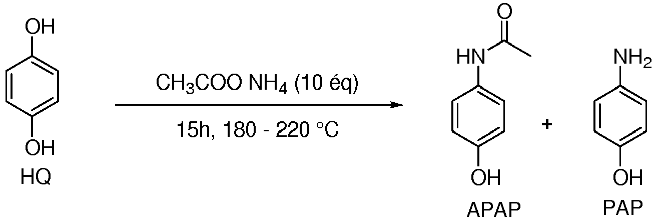

**Tableau 1 : Conversion de HQ, sélectivité en APAP et ratio APAP/PAP en fonction de la température de la réaction et de la présence d'eau.**

| Expérience | Ratio eau / HQ | Température | Conversion | Sélectivité APAP + PAP | Ratio APAP / PAP |
|---|---|---|---|---|---|
| 1 | - | 180 °C | 48% | 92% | 85/15 |
| 2 | - | 200 °C | 65% | 91% | 85/15 |
| 3 | - | 215 °C | 95% | 95% | 89/11 |
| 4 | - | 220 °C | 98% | >95% | 79/21 |
| 5 | 10 / 1 | 220 °C | 98% | >95% | 80/20 |

Pour des températures inférieures à 220 °C, la conversion augmente progressivement en fonction de la température (Tableau 1, entrées 1 à 3) : de 48% à 180 °C jusqu'à 95% à 215 °C. Une conversion quasi-totale a été obtenue à partir de 220 °C (Tableau 1, entrée 4).

De plus, les sélectivités APAP + PAP sont faiblement affectées par la température.

Enfin, la présence d'eau, introduite volontairement dans la réaction, ne perturbe ni la conversion (98% de conversion, identique sans eau), ni le ratio APAP/PAP (80/20 contre 79/21) (comparer Tableau 1, entrées 5 et 4).

### • Réaction en l'absence d'acide acétique - Influence du ratio hydroquinone / acétate d'ammonium sur la conversion et la sélectivité

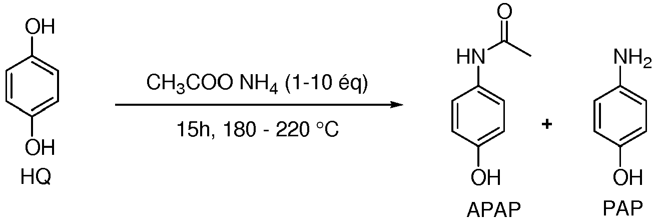

**Tableau 2 : Conversion de HQ, sélectivité en APAP et ratio APAP/PAP en fonction de la température de la réaction et du ratio acétate d'ammonium / hydroquinone**

| Expérience | Température | Ratio CH₃COONH₄/HQ | Conversion | Sélectivité APAP + PAP | Ratio APAP/PAP |
|---|---|---|---|---|---|
| 1 | 180 °C | 1 / 1 | 63% | 68% | 91/9 |
| 2 | | 3,3 / 1 | 58% | 94% | 87/13 |
| 3 | | 10 / 1 | 48% | >95% | 85/15 |
| 4 | 220 °C | 1 / 1 | 98% | 42% | 83/17 |
| 5 | | 10 / 1 | 96% | >95% | 79/21 |

Avec un seul équivalent d'acétate d'ammonium par rapport à l'hydroquinone, à 180 °C (Tableau 2, entrée 1), 63% de conversion et 68% de sélectivité en APAP + PAP sont obtenus alors que la sélectivité est >95% avec 10 équivalents d'acétate d'ammonium par rapport à l'hydroquinone (Tableau 2, entrée 3).

Cet effet est encore plus visible à 220 °C (Tableau 2, entrées 4 et 5), puisqu'à conversion presque égale, la sélectivité chute à 42% avec 1 équivalent d'acétate d'ammonium contre >95% avec 10 équivalents d'acétate d'ammonium.

Cette chute de la sélectivité en APAP + PAP lorsque le ratio acétate d'ammonium / hydroquinone est diminué peut s'expliquer par le fait qu'avec un seul équivalent d'acétate d'ammonium par rapport à l'hydroquinone, le milieu est plus concentré en HQ, en APAP et en PAP, ce qui favorise des réactions indésirables entre eux pour former principalement des diarylamines.

### • Réaction en présence d'acide acétique - Influence du ratio hydroquinone / acétate d'ammonium / acide acétique sur la conversion et la sélectivité

Dans ces expériences, la température de la réaction était de 220°C.

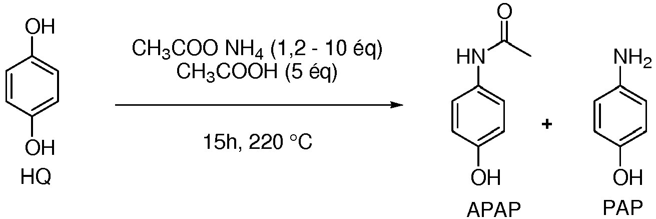

**Tableau 3 : Conversion de HQ, sélectivité en APAP et ratio APAP/PAP en fonction du ratio hydroquinone / acétate d'ammonium / acide acétique.**

| Expérience | Ratio HQ / AcONH₄ / AcOH | Conversion | Sélectivité APAP | ratio APAP / PAP |
|---|---|---|---|---|
| 1 | 1 / 10 / - | 96% | > 95% | 79/21 |
| 2 | 1 / 10 / 5 | 96% | > 95% | 100/0 |
| 3 | 1 / 2 / 5 | 85% | > 95% | 100/0 |
| 4 | 1 / 1,2 / 5 | 76% | > 95% | 100/0 |

Tout d'abord, afin de comparer avec les résultats des tableaux 1 et 2 qui précèdent, 5 équivalents d'acide acétique par rapport à l'hydroquinone ont été ajoutés en maintenant les autres conditions (ratio acétate d'ammonium / hydroquinone = 10/1 et température de la réaction de 220°C) identiques aux meilleures conditions mises en évidences dans les tableaux 1 et 2 (Tableau 3, entrées 1 et 2). La conversion reste la même (96%) avec ou sans acide acétique, mais le ratio APAP/PAP augmente : en présence de 10 équivalents d'acide acétique, l'acétylation du para-aminophénol est totale.

De plus, en réduisant le nombre d'équivalent d'acétate d'ammonium par rapport à l'hydroquinone, on observe toujours une acétylation totale du para-aminophénol, mais la conversion de l'hydroquinone baisse nettement : 85% avec 2 équivalents d'acétate (Tableau 3, entrée 3), et 76% avec 1,2 équivalent (Tableau 3, entrée 4).
Ceci pourrait s'expliquer par le fait qu'avec 10 équivalents d'acétate d'ammonium, l'acétate d'ammonium est plus disponible et sa réaction avec l'hydroquinone est plus rapide. Lorsque l'on baisse le nombre d'équivalents d'acétate d'ammonium en maintenant celui d'acide acétique, l'espèce majoritaire devient l'acide acétique : l'acétate d'ammonium est plus dilué, sa réaction avec l'hydroquinone est ralentie.

### • Exemple comparatif avec le brevet US 5,856,575

La réaction a été reproduite avec de l'acétamide comme réactif à la place de l'acétate d'ammonium, avec ou sans catalyseur hétéropolyacide.

Les réactions ont toutes été réalisées à une température de 220°C, en l'absence d'acide acétique.

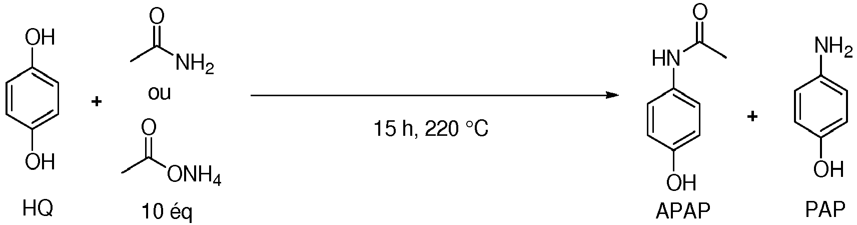

**Tableau 4 : Conversion de HQ, sélectivité en APAP et ratio APAP/PAP en fonction du réactif d'amidation utilisé (CH₃CONH ou CH₃COONH₄) et en présence ou en l'absence de catalyseur hétéropolyacide.**

| Expérience | Catalyseur hétéropolyacide | Réactif | Conversion (HQ) | Sélectivité APAP + PAP ou APAP | Ratio APAP / PAP |
|---|---|---|---|---|---|
| 1* (comparatif) | Acide phosphotungstique | CH₃CONH₂ (1 éq) | 95% | Sélectivité. APAP 86% | - |
| 2** (comparatif) | Acide silicotungstique | CH₃COONH₄ (1 éq) | 88% | Sélectivité. APAP 77% | - |
| 3 (comparatif) | - | CH₃CONH₂ (10 éq) | 27% | Sélectivité APAP + PAP 84% | 94/6 |
| 4 | - | CH₃COONH₄ (10 éq) | 96% | Sélectivité APAP + PAP > 95% | 79/21 |

| | | | | | |
|---|---|---|---|---|---|
| * D'après les résultats de l'exemple 2 de US 5,856,575 ** D'après les résultats de l'exemple 6 de US 5,856,575 | | | | | |

En présence de catalyseur hétéropolyacide, la conversion de hydroquinone de la réaction à partir d'acétamide (Tableau 4, entrée 1) est de 95%, alors que la conversion de hydroquinone de la réaction à partir d'acétate d'ammonium est de 88% (Tableau 4, entrée 2). En présence de catalyseur hétéropolyacide, l'acétate d'ammonium est donc moins réactif que l'acétamide.
En présence de catalyseur hétéropolyacide, les sélectivités en APAP+PAP sont meilleures en mettant en oeuvre la réaction avec de l'acétamide (86%, Tableau 4, entrée 1) qu'avec de l'acétate d'ammonium (77%, Tableau 4, entrée 2).

En l'absence de catalyseur hétéropolyacide, la conversion de l'hydroquinone de la réaction à partir d'acétamide (Tableau 4, entrée 3) est de 27%, alors que la conversion de l'hydroquinone de la réaction à partir d'acétate d'ammonium est de 96% (Tableau 4, entrée 4). En l'absence de catalyseur hétéropolyacide, l'acétamide est donc beaucoup moins réactif que l'acétate d'ammonium.
En l'absence de catalyseur hétéropolyacide, les sélectivités en APAP+PAP obtenues sont meilleures en mettant en oeuvre la réaction avec de l'acétate d'ammonium (>95%, Tableau 4, entrée 4) qu' avec de l'acétamide (84%, Tableau 4, entrée 3).

Les conversions de l'hydroquinone et les sélectivités en APAP+PAP sont donc différentes selon que la réaction est mise en oeuvre avec de l'acétamide ou avec de l'acétate d'ammonium, et avec ou sans catalyseur hétéropolyacide. La meilleure conversion de l'hydroquinone et la meilleure sélectivité en APAP+PAP ont été obtenues dans les conditions de l'invention (réaction mise en oeuvre avec de l'acétate d'ammonium, en l'absence de catalyseur).

### • Réaction à plus grande échelle (« scale-up ») en présence d'acide acétique et récupération du N-acétyl-p-aminophénol formé

La réaction a été réalisée dans un réacteur Parr d'une capacité de 300 mL. L'hydroquinone (44,3 g ; 0,40 mol) et l'acétate d'ammonium (63,0 g ; 0,80 mol) ont été introduits dans la cuve puis l'acide acétique (114 mL ; 1,99 mol) a été ajouté (proportions molaires HQ / AcOONH₄ / AcOH : 1 / 2 / 5). Le réacteur a été fermé et purgé sous flux d'argon pendant 15 min. Le réacteur a alors été mis en chauffe. La température a été suivie directement dans le réacteur. Une fois les 200 °C atteints, la réaction a été mise sous agitation (500 tr/min). Lorsque la température a atteint 230°C, la réaction a été laissée 15 h sous agitation.

L'analyse HPLC a montré une conversion de 93% de l'hydroquinone en N-acétyl-p-aminophénol.

Le N-acétyl-p-aminophénol a été récupéré comme suit. Le milieu (216 g) a été évaporé durant 30 min à 60 °C sous vide (8 mbar). 98 mL de l'acide acétique ont ainsi été récupérés (soit 85% de récupération). Après évaporation de l'acide acétique, 20 mL d'eau ont été ajouté au milieu (masse : 109 g). Puis le milieu a été laissé à température ambiante (environ 25°C) durant 30 min. Le précipité formé a alors été filtré sur fritté et le ballon a été rincé avec 2 x 20 mL d'eau froide. Le précipité récupéré a ensuite été lavé avec 2 x 20 mL d'eau froide (à une température entre 10 et 15°C). On a ainsi obtenu 53,2 g d'APAP brut (rendement de 88%, soit 95% de récupération de l'APAP formé). Le produit a été obtenu avec une pureté de 99% (1 % d'hydroquinone reste dans le produit final et quelques traces d'acétamide).
Le N-acétyl-p-aminophénol peut alors être recristallisé dans l'eau si une pureté supérieure à 99% est souhaitée.

La conversion de l'hydroquinone et la sélectivité en N-acétyl-p-aminophénol sont donc améliorées par rapport au procédé de préparation de N-acétyl-p-aminophénol par réaction de l'hydroquinone avec l'acétamide en présence d'un catalyseur hétéropolyacide décrit dans le brevet US 5,856,575.

### • Réaction à partir d'un mélange d'acétate d'ammonium et d'acétamide en présence d'eau

Lorsque l'eau du filtrat obtenu lors de la filtration de l'APAP est évaporée, on obtient un résidu qui comprend principalement de l'hydroquinone, de l'acétate d'ammonium et de l'acétamide. L'utilisation de ce résidu dans la réaction est avantageuse en ce que l'hydroquinone et l'acétate d'ammonium qu'il contient sont les réactifs de la réaction selon l'invention. Par contre, le résidu comprend également de l'acétamide. L'objectif de cet exemple était de vérifier que, lorsque la réaction est mise en oeuvre en présence d'eau, l'acétamide s'hydrolyse *in situ* pour former l'acétate d'ammonium, ce qui démontre la faisabilité du recyclage du résidu, sans enrichir le milieu réactionnel en acétamide.

La réaction a été réalisée à partir d'un mélange 1/1/1/1/5 d'acétate d'ammonium / acétamide / eau / hydroquinone / acide acétique et les résultats (tableau 5 entrée 2) ont été comparé à ceux obtenus avec une réaction réalisée à partir d'un mélange 2/1/5 d'acétate d'ammonium / hydroquinone / acide acétique (en l'absence d'acétamide et en l'absence d'eau) (tableau 5 entrée 1). Les réactions ont été réalisées à une température de 220°C.

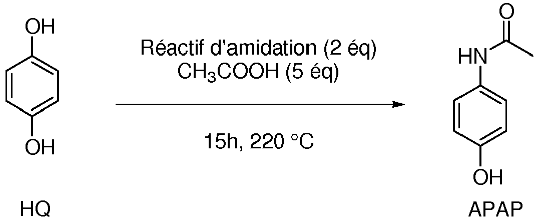

**Tableau 5 : Conversion de HQ, sélectivité en APAP et ratio APAP/PAP en fonction du réactif d'amidation utilisé**

| Expérience | Réactif | Conversion (HQ) | Sélectivité APAP + PAP | Ratio APAP / PAP |
|---|---|---|---|---|
| 1 | CH₃COONH₄ (2 éq) | 85% | >95% | 100/0 |
| 2 | CH₃COONH₄ / CH₃COONH₂ / H₂O (1/1/1 éq) | 82% | >95% | 100/0 |

Les conversions de l'hydroquinone et les sélectivités en APAP + PAP sont similaires (Tableau 5, entrées 1 et 2), ce qui confirme que l'acétamide s'hydrolyse en acétate d'ammonium. Il est donc bien possible de recycler le résidu obtenu dans la réaction.

## Revendications

1. Procédé de préparation de N-acétyl-para-aminophénol comprenant une étape de réaction de l'hydroquinone avec l'acétate d'ammonium, la réaction étant réalisée en l'absence d'hétéropolyacide ou d'un de ses sels.

2. Procédé selon la revendication 1, dans lequel la réaction est réalisée en l'absence de solvant.

3. Procédé selon la revendication 2, dans lequel au moins 2, notamment au moins 3, de préférence au moins 10 équivalents d'acétate d'ammonium par rapport à l'hydroquinone sont utilisés.

4. Procédé selon la revendication 1, dans lequel la réaction est réalisée en présence d'acide acétique.

5. Procédé selon la revendication 4, dans lequel de 1 à 10 équivalents, de préférence de 1 à 5 équivalents d'acide acétique par rapport à l'hydroquinone sont utilisés.

6. Procédé selon la revendication 4 ou 5, dans lequel au moins 1,1, notamment au moins 1,2, de préférence au moins 2 équivalents d'acétate d'ammonium par rapport à l'hydroquinone sont utilisés.

7. Procédé selon l'une quelconque des revendications 1 à 6, dans lequel la réaction est réalisée à une température de 150°C à 300°C, notamnent de 200 à 250°C, de préférence de 215 à 230 °C.

8. Procédé selon l'une quelconque des revendications 1 à 7, dans lequel la réaction est réalisée en l'absence de catalyseur métallique.

9. Procédé selon l'une quelconque des revendications 1 à 8, comprenant une étape ultérieure de récupération du N-acétyl-para-aminophénol.

10. Procédé selon la revendication 9, dans laquelle l'étape de récupération comprend les étapes de :
a) ajout d'eau au milieu réactionnel obtenu à la fin de la réaction, ce par quoi le N-acétyl-para-aminophénol précipite,
b) filtration du N-acétyl-para-aminophénol,
c) éventuellement recristallisation du N-acétyl-para-aminophénol dans l'eau.

11. Procédé selon la revendication 10, dans lequel la réaction de l'hydroquinone avec l'acétate d'ammonium est réalisée en présence d'acide acétique et dans lequel l'étape de récupération comprend, avant l'étape a), une étape α) d'évaporation de l'acide acétique du milieu réactionnel obtenu à la fin de la réaction.

12. Procédé selon la revendication 11, dans lequel l'acide acétique évaporé est récupéré et recyclé dans la réaction de l'hydroquinone avec l'acétate d'ammonium.

13. Procédé selon l'une quelconque des revendications 10 à 12, comprenant après l'étape b) des étapes de :
d) lavage du filtrat obtenu à l'étape b) avec un solvant apolaire,
e) évaporation de l'eau du filtrat pour obtenir un résidu,
f) introduction du résidu obtenu dans le milieu réactionnel lors de la réaction de l'hydroquinone avec l'acétate d'ammonium.

## Patentansprüche

1. Verfahren zur Herstellung von N-Acetyl-para-Aminophenol, umfassend einen Reaktionsschritt des Hydrochinon mit Ammoniumacetat, wobei die Reaktion unter Ausschluss von Heteropolysäure oder einem ihrer Salze realisiert wird.

2. Verfahren nach Anspruch 1, bei dem die Reaktion unter Ausschluss eines Lösungsmittels realisiert wird.

3. Verfahren nach Anspruch 2, bei dem mindestens 2, insbesondere mindestens 3, vorzugsweise mindestens 10 Äquivalente des Ammoniumacetats in Bezug auf das Hydrochinon verwendet werden.

4. Verfahren nach Anspruch 1, bei dem die Reaktion in Anwesenheit von Essigsäure realisiert wird.

5. Verfahren nach Anspruch 4, bei dem 1 bis 10 Äquivalente, vorzugsweise 1 bis 5 Äquivalente der Essigsäure in Bezug auf das Hydrochinon verwendet werden.

6. Verfahren nach Anspruch 4 oder 5, bei dem mindestens 1,1, insbesondere mindestens 1,2, vorzugsweise mindestens 2 Äquivalente des Ammoniumacetats in Bezug auf das Hydrochinon verwendet werden.

7. Verfahren nach einem beliebigen der Ansprüche 1 bis 6, bei dem die Reaktion bei einer Temperatur von 150°C bis 300°C, insbesondere von 200 bis 250°C, vorzugsweise von 215 bis 230°C realisiert wird.

8. Verfahren nach einem beliebigen der Ansprüche 1 bis 7, bei dem die Reaktion in Abwesenheit eines Metallkatalysators durchgeführt wird.

9. Verfahren nach einem beliebigen der Ansprüche 1 bis 8, einen späteren Schritt der Rückgewinnung von N-Acetyl-para-Aminophenol umfassend.

10. Verfahren nach Anspruch 9, bei dem der Schritt der Rückgewinnung die Schritte umfasst:
a) Zufügen von Wasser zu dem am Ende der Reaktion erhaltenen Reaktionsmedium, wodurch das N-Acetyl-para-Aminophenol ausfällt,
b) Filtern des N-Acetyl-para-Aminophenols,
c) gegebenenfalls Rekristallisieren des N-Acetyl-para-Aminophenols in Wasser.

11. Verfahren nach Anspruch 10, bei dem die Reaktion des Hydrochinon mit Ammoniumacetat bei Vorhandensein einer Essigsäure durchgeführt wird und bei dem der Schritt der Rückgewinnung vor dem Schritt a) einen Schritt α) der Verdampfung der Essigsäure des am Ende der Reaktion erhaltenen Reaktionsmediums umfasst.

12. Verfahren nach Anspruch 11, bei dem die verdampfte Essigsäure wiedergewonnen und in der Reaktion des Hydrochinons mit dem Ammoniumacetat recycelt wird.

13. Verfahren nach einem beliebigen der Ansprüche 10 bis 12, nach dem Schritt b) die Schritte umfassend:
d) Waschen des bei Schritt b) erhaltenen Filtrats mit einem apolaren Lösungsmittel,
e) Verdampfen des Wassers des Filtrats, um einen Rückstand zu erhalten,
f) Zuführen des erhaltenen Rückstands in das Reaktionsmedium während der Reaktion des Hydrochinons mit dem Ammoniumacetat.

## Claims

1. Process for the preparation of N-acetyl-para-aminophenol, comprising a step of reacting hydroquinone with ammonium acetate, the reaction being carried out in the absence of heteropoly acid or a salt thereof.

2. Process according to claim 1, wherein the reaction is carried out in the absence of solvent.

3. Process according to claim 2, wherein at least 2, especially at least 3, preferably at least 10 equivalents of ammonium acetate relative to hydroquinone are used.

4. Process according to claim 1, wherein the reaction is carried out in the presence of acetic acid.

5. Process according to claim 4, wherein from 1 to 10 equivalents, preferably from 1 to 5 equivalents, of acetic acid relative to hydroquinone are used.

6. Process according to claim 4 or 5, wherein at least 1.1, especially at least 1.2, preferably at least 2 equivalents of ammonium acetate relative to hydroquinone are used.

7. Process according to any one of claims 1 to 6, wherein the reaction is carried out at a temperature of from 150°C to 300°C, especially from 200 to 250°C, preferably from 215 to 230°C.

8. Process according to any one of claims 1 to 7, wherein the reaction is carried out in the absence of metallic catalyst.

9. Process according to any one of claims 1 to 8, comprising a subsequent step of recovering N-acetyl-para-aminophenol.

10. Process according to claim 9, wherein the recovery step comprises the steps of:
a) adding water to the reaction mixture obtained at the end of the reaction, whereby the N-acetyl-para-aminophenol precipitates,
b) filtering the N-acetyl-para-aminophenol,
c) optionally recrystallising the N-acetyl-para-aminophenol in water.

11. Process according to claim 10, wherein the reaction of hydroquinone with ammonium acetate is carried out in the presence of acetic acid, and wherein the recovery step comprises, prior to step a), a step α) of evaporating off the acetic acid from the reaction mixture obtained at the end of the reaction.

12. Process according to claim 11, wherein the acetic acid that is evaporated off is recovered and recycled in the reaction of hydroquinone with ammonium acetate.

13. Process according to any one of claims 10 to 12, comprising after step b) steps of:
d) washing the filtrate obtained in step b) with an apolar solvent,
e) evaporating off the water from the filtrate in order to obtain a residue,
f) introducing the residue obtained into the reaction mixture during the reaction of hydroquinone with ammonium acetate.
